# EUROPEAN PATENT APPLICATION

(11) **EP 4 397 324 A1**
(43) Date of publication of application: **10.07.2024**
(21) Application number: 23150322.8
(22) Date of filing: 04.01.2023
(51) Int. Cl.: A61L 2/20, A61L 2/24, A61L 9/015

(54) **DECONTAMINATION SYSTEM HAVING A MODULAR DECONTAMINATION UNIT**

(71) Applicant: Halton OY, 47400 Kausala (FI)
(72) Inventor: Hagström, Kim, 47400 Kausala (FI); Grönvall, Ismo, 47400 Kausala (FI); Purho, Harri, 47400 Kausala (FI)
(74) Representative: Papula Oy

(57) **Abstract**

A decontamination system for sterilizing a contaminated space, comprising at least one decontamination unit (1) through which the air of the contaminated space is recirculated, which unit comprises
- an inlet arrangement (6) for introducing air from the contaminated space into the unit,
- a discharge arrangement (7) for discharging gas out of the unit,
- a fan (9) for creating an air flow from the inlet arrangement to the discharging arrangement,
- H₂O₂ supply device (10) for introducing H₂O₂ into the air flow inside the unit,
**characterized** in that
the decontamination unit comprises at least one of
- the discharge arrangement comprises detachable at least one outlet module (21) through which the H₂O₂-air mixture is discharged out of the unit, and
- the inlet arrangement comprises at least one detachable inlet module (20) for receiving air from a space into the unit.

## Description

### TECHNICAL FIELD

The present invention relates to apparatuses decontaminating space such as hospital room or other clean room.

### BACKGROUND OF THE ART

Clean rooms and other critical spaces, such as manufacturing facilities in food and pharmaceutical industries may be exposed to unwanted particles and impurities, such as mold and unwanted microbes, and the spaces may be contaminated. Before operation may begin or continue, the spaces must be decontaminated. Another example where decontamination is needed is military industry. Military equipment may be exposed or contaminated with biological active substances such as biological or chemical warfare agents, such as pathogens, biotoxins, prions, chemical agents (e.g. nerve gas) etc.

Vaporized solution of hydrogen peroxide (H₂O₂) has been used to decontaminate the internal surfaces of enclosures used for aseptic processing in the pharmaceutical industry since about 1990, but it has always been difficult to use the same technology to decontaminate larger enclosed volumes such as rooms. There have been some apparatuses which uses separate H₂O₂ pumps for spraying H₂O₂ gas into a space or spraying H₂O₂ gas together with an air stream into a space.

The known solutions are complicated and built so that only limited low carrier air flow may be used to evaporate H₂O₂. The amount of air is insufficient to properly diffused the mixture within the space. Another disadvantage is that the H₂O₂ is insufficiently mixed with the carrier gas prior introduction to the decontaminated space. Thus, there is a risk for H₂O₂ condensation on the surfaces within the apparatus and within the decontaminated space causing risk of material failure. Further, some systems are complicated and, thus, costly apparatuses using separate fans, heating, drying, and/or filtering for providing proper diffusion of H₂O₂ into the decontaminated space.

Further, known devices have been designed to certain applications and different kinds of devices have been used for different scenarios.

### OBJECTIVE OF THE INVENTION

The objective of the device is to alleviate the disadvantages mentioned above.

In particular, it is an objective of the present device to provide a decontamination unit which may be used in different scenarios.

### SUMMARY

According to a first aspect, the present invention provides a decontamination system comprising modular decontamination unit of different decontamination situations.

According to a first aspect, the invention provides a decontamination system for sterilizing a contaminated space, comprising at least one decontamination unit through which the air of the contaminated space is recirculated. The unit comprises
- an inlet arrangement for introducing air from the contaminated space into the unit,
- a discharge arrangement for discharging gas out of the unit,
- a fan for creating an air flow from the inlet arrangement to the discharging arrangement, and
- H₂O₂ supply device for introducing H₂O₂ into the air flow inside the unit.
The decontamination unit comprises at least one of
- the discharge arrangement comprises at least one detachable outlet module through which the H₂O₂-air mixture is discharged out of the unit, and
- the inlet arrangement comprises at least one detachable inlet module for receiving air from a space into the unit.

The advantage of the system is that unit may be used in different decontamination situations, i.e. in same space of the contamination or placed on different location to the contaminated space.

According to an embodiment, the discharge arrangement comprises the detachable outlet module comprising a diffuser.

According to an embodiment, the diffuser comprises multiple mini diffusers for controlling the diffuser pattern.

According to an embodiment, the discharge arrangement comprises the detachable outlet module comprising at least one tube connector for connecting the outlet chamber into air channel, such as tube or duct, for supplying the H₂O₂-air mixture from the decontamination unit into the air channel.

According to an embodiment, the discharge arrangement comprises the detachable outlet module comprising at least one clamp connector for connecting the unit to ductwork or a hole in wall of a space, which allows diffusing the H₂O₂-air mixture either directly into the space, or to a duct or tube.

According to an embodiment, the system comprises at least two detachable outlet modules of group: outlet module with diffuser, outlet module with tube connector, and outlet module with clamp connector.

According to an embodiment, the inlet arrangement comprises the detachable inlet module comprising a grille or perforated plate.

According to an embodiment, the inlet arrangement comprises the detachable inlet module comprising at least one tube connector for connecting the unit to an air channel, such as a tube or a duct, for receiving air via air channel from different location.

According to an embodiment, the inlet arrangement comprises the detachable inlet module comprising at least one clamping ring for connecting the unit to ductwork or a hole in wall of a space, which allows receiving the air either directly from the space, or via a duct or tube.

According to an embodiment, the inlet arrangement comprises control means for channeling the air in different air chambers inside the unit.

According to an embodiment, the inlet arrangement comprises at least two openings, wherein at least two openings lead to different air chambers inside the unit.

According to an embodiment, the control means comprises a damper arranged at the at least two openings for controlling into which air chamber the air from the contaminated space is introduced.

According to an embodiment, the system comprises tubing connected to the outlet module, which tubing ends to another space than the location of the decontamination unit.

According to an embodiment, the system comprises tubing connected to the inlet module, which tubing ends to another space wherein the unit is located.

According to an embodiment, the unit comprises an inlet chamber for receiving the air from the contaminated space and a filtering chamber for removing H₂O₂ from the air from the space.

It is to be understood that the aspects and embodiments of the invention described above may be used in any combination with each other. Several of the aspects and embodiments may be combined together to form a further embodiment of the invention.

### BRIEF DESCRIPTION OF THE DRAWINGS

The accompanying drawings, which are included to provide a further understanding of the invention, and constitute a part of this specification, illustrate embodiments of the invention and together with the description help to explain the principles of the invention. In the drawings:
**Fig. 1a and 1b** show an embodiment of inlet arrangement and discharge arrangement,
**Fig. 2a and 2b** show similar unit as in figure 1a and 1b, but the inlet arrangement comprises tube connectors, and
**Fig. 3a and 3b** show the control means for channeling the air from the contaminated space in different chambers in more detail,
**Fig. 4a and 4b** show a decontamination unit comprising a frame having an inner space defining the enclosure inside the unit,
**Fig. 5a and 5b** show an embodiment, wherein the inner space comprises a filtering chamber having H₂O₂ capturing means, and
**Fig. 6a and 6b** show an embodiment wherein the second inlet is arranged to a separate plate.

### DETAILED DESCRIPTION

Figures show a decontamination system comprising a decontamination unit for sterilizing a space, such as a contaminated room or other contaminated space, with H₂O₂. The decontamination unit is capable to recirculate the air of the space. For decontaminating a space, the space must be first sterilized with H₂O₂ and then the H₂O₂ must be removed from the space. With the present decontamination system, this may be done with separate sterilizing cycle and flushing cycle. In sterilizing cycle and in flushing cycle the air is recirculated inside the unit via different routes. The H₂O₂ is introduced into the air flow inside the unit by H₂O₂ supply device. The air flow is shown in figures as hollow arrows.

The present decontamination unit of the system comprises discharging arrangement comprising at least one detachable outlet module. The detachable outlet module may comprise different kind of connectors for connecting the unit to air channel, such as tube, duct, or opening in a wall; or diffuser for discharging recirculated air out of the unit. The outlet module may be replaced with another outlet module if different kind of connection is needed. The air is recirculated by a fan creating an air flow from the inlet arrangement to the discharging arrangement. The different outlet modules may be provided at different locations in the decontamination unit, e.g. at the top of the unit and/or at side wall of the unit.

The discharging arrangement may be positioned at a side wall of the unit or at top of the decontamination unit.

For introducing the air into the decontamination unit, the unit comprises inlet arrangement comprising a grille, perforated plate, or opening(s) for introducing air from the contaminated space into the decontamination unit. Optionally, the inlet arrangement may comprise detachable module comprising grille, perforated plate, or opening(s). Optionally, the inlet arrangement comprises at least one tube connector or clamp connector for connecting the unit to an air channel. Optionally, the inlet arrangement comprises detachable inlet module comprising at least one tube connector or clamp connector for connecting the unit to an air channel. Optionally, the inlet arrangement comprises inlets for channeling the air to different chambers inside the decontamination unit. The inlet arrangement may be positioned at the side wall of the decontamination unit.

For example, the unit may be placed directly inside a contaminated space, e.g. room, and the air of the contaminated space is recirculated through the decontamination unit through the inlet arrangement and the discharging arrangement. The air from the contaminated space is introduced directly from the space into the unit and H₂O₂-air mixture is discharged out of the unit directly into the contaminated space. The inlet arrangement may comprise a grille, perforated plate, or opening(s) for introducing air from the contaminated space into the decontamination unit. Optionally, the inlet may comprise detachable inlet module comprising grille, perforated plate, or opening(s).

In some situations, the decontamination unit is placed to another location and the contaminated space is elsewhere. The outlet module may comprise some connection means, e.g. tube connector(s) or clamp connector(s) for connecting the unit to some air channel (s) for discharging the H₂O₂-air mixture to the contaminated space, which may be elsewhere, via the air channel (s). The inlet arrangement may comprise a grille, perforated plate, or opening(s) for introducing air from the contaminated space into the decontamination unit. Optionally, the inlet arrangement may comprise detachable module comprising grille, perforated plate, or opening(s).

In another situation, the air to the decontamination unit is located inside the contaminated space but the air into the unit is sucked from some other space. Thus, the inlet arrangement may comprise detachable inlet module, which may be replaced with another inlet module. For example, the detachable inlet module may comprise tube connector(s) or clamp connector(s) for connecting the unit to an air channel(s) for introducing air into the decontamination unit via the air channel (s). The air channel (s) end may be in other space than the unit. The discharging arrangement may comprise at least one opening or a diffuser for discharging H₂O₂-air mixture directly into the space, where the decontamination unit is placed. The opening (s) or diffuser may be arranged on the detachable outlet module of the discharging arrangement.

In some situations, the decontamination unit is placed to another location and the contaminated space is elsewhere. The outlet module may comprise some connection means, e.g. tube connector(s) or clamp connector(s) for connecting the unit to some air channel (s) for discharging the H₂O₂-air mixture to the contaminated space, which may be elsewhere, via the air channel(s). The air into the unit is sucked from some other space. Thus, the arrangement may comprise tube connector(s) or clamp connector(s) for connecting the unit to an air channel(s) for introducing air into the decontamination unit via the air channel(s). Optionally, the inlet arrangement may comprise detachable inlet module, which may be replaced with another inlet module. For example, the detachable inlet module may comprise tube connector(s) or clamp connector(s) for connecting the unit to an air channel(s) for introducing air into the decontamination unit via the air channel (s). The air channel (s) end may be in other space than the unit.

Optionally, at least one of the detachable outlet module and detachable inlet module comprises two or more openings. The outlet module comprising multiple openings may be configured to discharge H₂O₂-air mixture either directly into the space, in which the decontamination unit is placed, via one opening, or into another space via air channels connected to another opening. Optionally, the inlet arrangement may comprise a grille, perforated plate, or opening(s) and a tube connector(s) or clamp connector(s) for introducing air from the contaminated space into the decontamination unit. Optionally, the inlet arrangement may comprise detachable module comprising grille, perforated plate, or opening(s).

The system may comprise at least two detachable outlet modules of group: outlet module with diffuser, outlet module with tube connector, and outlet module with clamp connector. Outlet modules may be replaced with another and used one at a time for different situations.

The inlet arrangement of the decontamination unit may comprise control means for channeling the air in different air chambers inside the unit. The control means may comprise a damper arranged at the at least two openings for controlling into which air chamber the air from the contaminated space is introduced. Optionally, the inlet arrangement comprises at least two openings, wherein at least two openings lead to different air chambers inside the unit. For example, the unit may comprise an inlet chamber for receiving the air from the contaminated space and a filtering chamber for removing H₂O₂ from the air from the space.

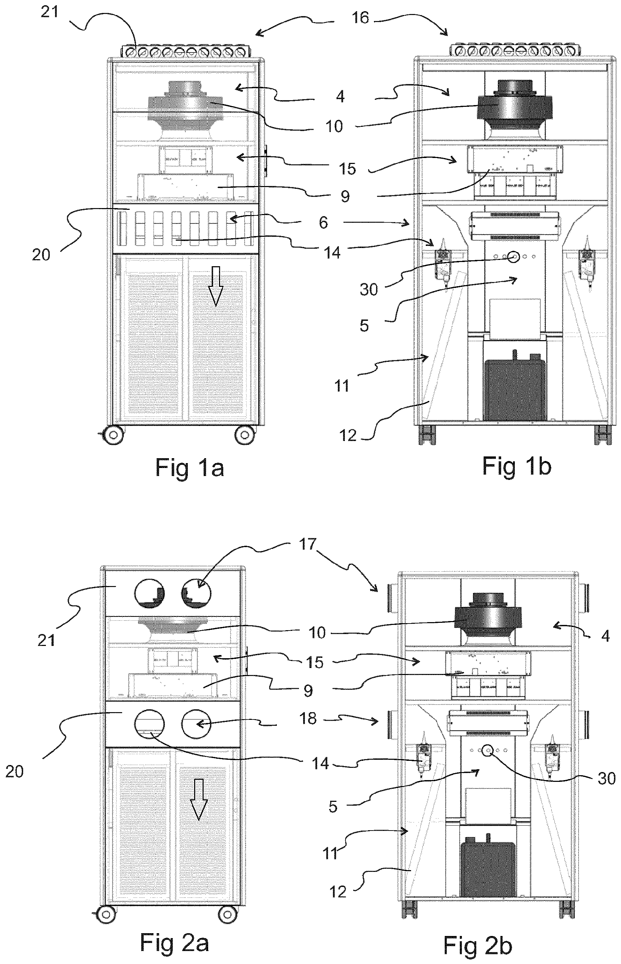

Figures 1a and 1b show some options of the discharging arrangements and inlet arrangement. The inlet arrangement 6 comprises detachable inlet module 20 comprising a perforated plate 19 through which the air from the contaminated space is introduced into the decontamination unit 1. The inlet arrangement comprises a separate plate after the perforated plate and the separate plate comprises two inlets, one for the inlet chamber 5 and one for the filtering chamber 11 for channeling the air different routes during different decontamination cycles. The inlet arrangement comprises control means 14 at the inlets for channeling the air. The control means comprises a damper or dampers or other adjustable or slidable plate. In figures 1a and 1b the channeling is achieved by using a damper at each inlet, which dampers may be rotated about an axis to open or close the inlets for channeling the air into the desired chamber. In these figures, a damper covers the inlet which leads directly into the inlet chamber 5. Thus, the unit is shown in flushing cycle, and the air is channeled through the filtering chamber 11. The recirculated air is discharged out of the unit via discharging arrangement comprising diffuser 16 having multiple mini diffusers.

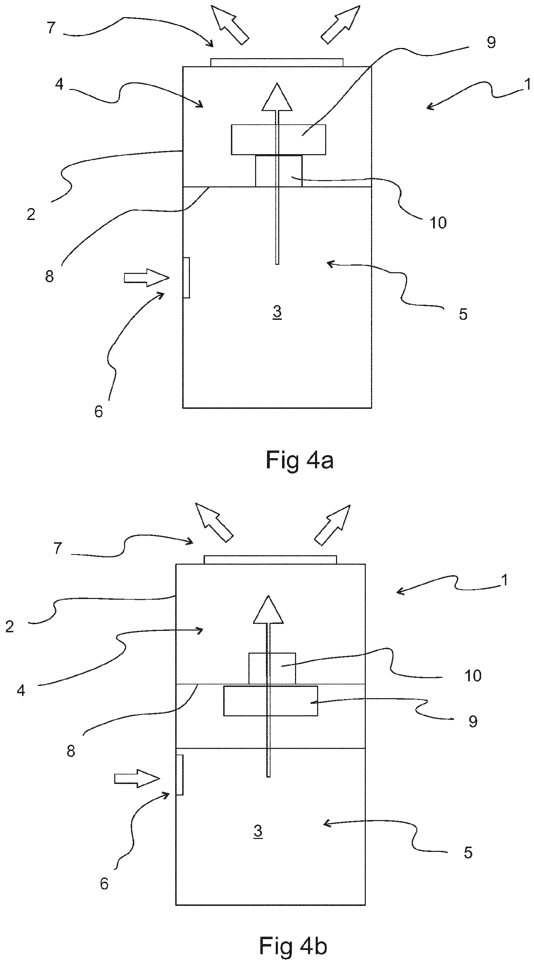

Figures 2a and 2b show similar unit as in figure 4a and 4b, but the inlet arrangement 6, through which the air from the contaminated space is introduced into the decontamination unit 1, comprises two tube connectors 18 for connecting the unit to tubes or ducts. Also, the discharging arrangement comprises detachable outlet module comprising tube connectors 17 for discharging the H₂O₂-air mixture to another space via tubes or ducts connected to the unit.

As shown in figures 1a, 1b, 2a and 2b, the decontamination unit 1 may comprise several inlet arrangements and discharging arrangements on different sides of the unit.

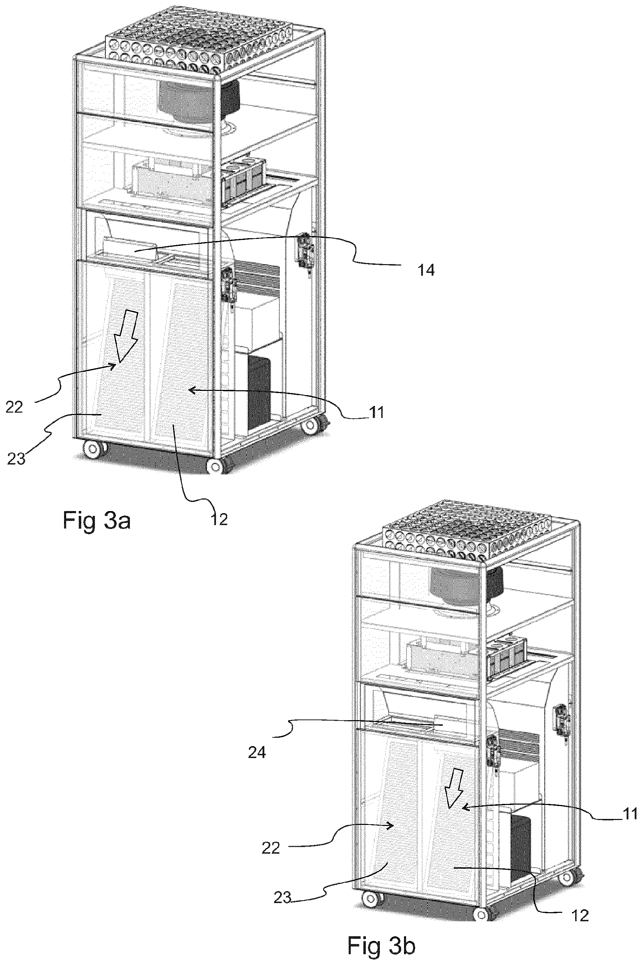

Figure 3a and 3b show the inlet arrangement as an opening and control means for channeling the air from the contaminated space in different chambers in the decontamination unit 1. In figure 3a, the damper 14 is in an open position and the inlet that leads directly into the inlet chamber 5 is open, and the air is flown to the inlet chamber 5 without circulating through the filtering chamber 11, i.e. the unit is shown in sterilizing cycle. In figure 3b, another damper 24 is in open position and the inlet that leads to the filtering chamber 11, and the air is flown to the filtering chamber 11, i.e. the unit is shown in the flushing cycle. The H₂O₂-air mixture is discharged through discharge arrangement comprising a diffuser with multiple mini diffusers.

The decontamination unit may comprise filtering means for filtering dust or other unwanted particles from entering the unit. The filtering means may comprise at least one filter. For example, a filter 23 may be arranged between the inlet, through which the air from the contaminated space is introduced into the unit,and the inlet chamber 5, as seen for example in figures 3a and 3b. The filter may be arranged at an inlet or inside the inner space.

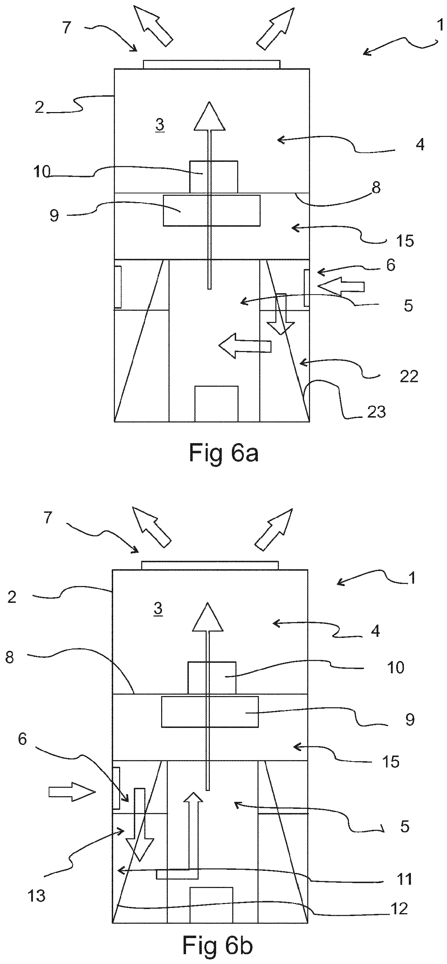

The decontamination unit may comprise a mid-chamber 22 arranged adjacent to the filtering chamber 11. The mid-chamber 22 may be located between the inlet, through which the air from the contaminated space is introduced into the unit, and the inlet chamber. The filtering means may be arranged between the mid-chamber 22 and the inlet chamber 5. The mid-chamber 22 and the filtering chamber 11 may be separated by a plate for preventing unwanted air flow between the chambers. The optional mid-chamber is shown for example in figures 3a,3b and 6a.

The inlet of the decontamination unit may comprise an entry chamber and an opening, through which the air is introduced from the space into the unit. The opening is arranged at the wall of the frame and the entry chamber is between the opening and the inlets, through which the air from the room is channeled to the inlet chamber (either directly or via mid-chamber) or to the filtering chamber.

It should be noted that the different inlet arrangements and discharging arrangement described herein are suitable to be used with different inner space 3 arrangements described herein. For example, different discharge arrangements (diffuser(s), clamp connector, tube connector etc.) or inlet arrangements are described herein together with specific air chamber arrangements, they may be provided with units having different air chamber arrangements, e.g. which air chambers (filtering chamber 11, H₂O₂ generator chamber 11 etc.) is provided. Also, different kind of discharge arrangements with detachable outlet module and inlet arrangements with detachable inlet modules may be implemented to different embodiments, although not described together with all embodiments.

The following describes decontamination unit options for the system for ensuring sufficient H₂O₂-air mixture diffusion into the contaminated space and flushing the space after sufficient H₂O₂ dose in the space is reached.

Figure 4a and 4b show a decontamination unit 1 comprising a frame 2 having an inner space 3 defining the enclosure inside the unit. The inner space 3 is divided in two air chambers, which are an outlet chamber 4 and an inlet chamber 5. A first plate 8, having an opening for discharging air from the inlet chamber 5 into the outlet chamber 4, is provided between the inlet chamber 5 and the outlet chamber 4. The unit comprises at least one inlet 6 through which the air from the contaminated space is introduced into the and an outlet 7 having discharging arrangement for discharging gas mixture out of the decontamination unit 1. For creating an air flow from at least one inlet arrangement 6 to the discharging arrangement 7, a fan 10 is provided inside the unit. The fan 10 comprises a fan inlet for receiving air flow, and a fan for discharging the air flow. The fan 10 is arranged to the opening of the first plate 8 so that the air flow is flown through the opening. The unit comprises an H₂O₂ supply device 9 which is arranged next to the fan inlet or the fan outlet for introducing H₂O₂ directly into the air flow for forming H₂O₂-air mixture. In figure 4a, the H₂O₂ supply unit is arranged inside the outlet chamber 4 next to the fan outlet, i.e. after the fan 10 in air flow direction. The H₂O₂ supply device 9 may be directly connected to the fan 10, or a gap may be provided between the fan 10 and the H₂O₂ supply device 9. The gap, i.e. the distance between the fan and the H₂O₂ supply unit, may be for example 5 to 200 mm. In figure 1b, the H₂O₂ supply unit is arranged next to the fan inlet, i.e. before the fan 10 in air flow direction. The H₂O₂ supply device 9 may be directly connected to the fan 10, or a gap may be provided between the fan 10 and the H₂O₂ supply device 9. The gap, i.e. the distance between the fan and the H₂O₂ supply unit, may be for example 50 to 250 mm. Figure 4b shows also an optional H₂O₂ generator chamber, which will be discussed later. It should be noted that different locations of the H₂O₂ supply device 9 shown in figures 4a and 4b may be implemented to any embodiments described herein.

By arranging the H₂O₂ supply device 9 next to the fan 10, the H₂O₂ may be introduced directly into the air flow and a homogenous H₂O₂-air mixture may be provided that is effectively distributed to the decontaminated space and allowing faster decontamination cycle even in large and complicated spaces with just one decontamination unit 1. There is no need for additional air treatment, e.g. heating, drying, humidifying etc, or additional air circulating arrangement or fans within the decontamination unit.

The H₂O₂ supply device 9 may be for example H₂O₂ generator.

At least one inlet arrangement 6 is arranged so that the air from the contaminated space may be sucked into the inlet chamber 5 of the unit. The inlet arrangement 6 may be arranged to a side wall of the inlet chamber 5. Optionally, there are two, or more, inlet arrangements 6 for allowing suction of the air from the contaminated space into the inlet chamber 5. The separate inlet arrangements 6 may be provided at different side walls of the inlet chamber 5. The inlet arrangement or inlet arrangements 6 may be at the side wall of the frame 2, or they may be at some other plate or wall inside the inner space 3 for allowing suction of the contaminated air into the inlet chamber 5. For example, a separate wall may be provided inside the inner space 3, which separate wall is between the inlet chamber 5 and the frame 2 in air flow direction. Other inlet arrangements may be provided for introducing air into other chambers.

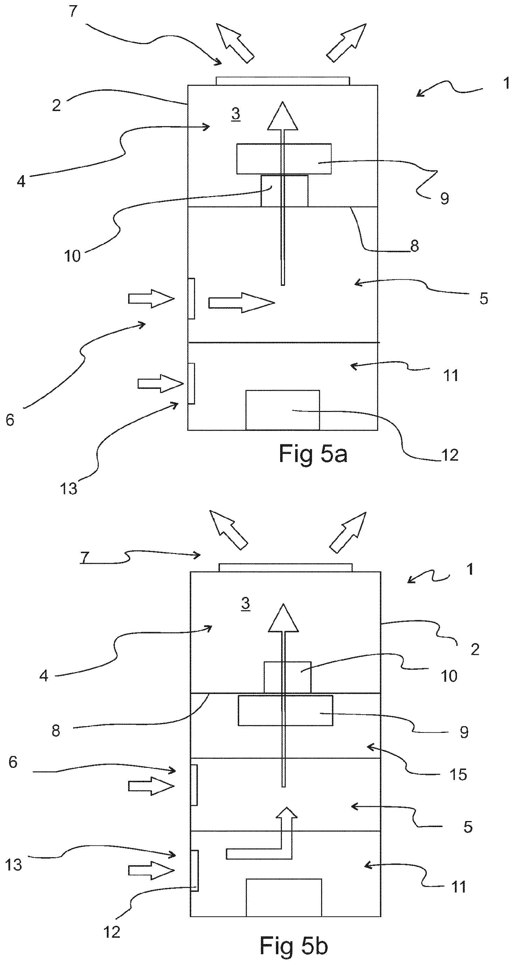

Figure 5a and 5b show an embodiment, wherein the inner space 3 comprises a filtering chamber 11 having H₂O₂ capturing means 12. In sterilizing cycle, as shown in figure 2a, the contaminated air is introduced to the inlet chamber 5 via at least one inlet arrangement 6, and is recirculated through inlet chamber 5, optional H₂O₂ generator chamber 15, and outlet chamber 4 before discharged back to the contaminated space via outlet arrangement 7. The unit may comprise at least one second inlet arrangement 13 for allowing suction of the air from the contaminated space into the filtering chamber 11. In flushing cycle, as shown in figure 2b, the air from the contaminated space is introduced to the filtering chamber 11 via the second inlet arrangement 13 and the H₂O₂ is captured from the air by H₂O₂ capturing means 12. The second inlet arrangement 13 may be at the wall of the frame 2, or it may be inside the inner space 3 so that it allows channeling the air flow from the inlet arrangement on the frame to the filtering chamber 11. The second inlet arrangement 13 may be arranged to a side wall of the filtering chamber 11. Optionally, there are two or more, second inlet arrangements 13 for allowing suction of the air from the contaminated space into the inlet chamber 5. The separate second inlet arrangements may be provided at different side walls of the frame 2, e.g. different sides of the filtering chamber 11. The second inlet arrangement 13 or inlet arrangements may be at the side wall of the frame 2, or they may be at some other plate or wall inside the inner space 3 for allowing suction of the air into the inlet chamber 5. For example, a separate wall may be provided inside the inner space 3, which separate wall is between the filtering chamber 11 and the frame 2 in air flow direction. The H₂O₂ capturing means 12 may comprise a filter or filters for removing H₂O₂ from the air from the space. The filter may be a catalytic filter. The catalytic substance may be for example potassium permanganate (KMnO₄). The substance may be impregnated to a fibrous or porous base material of the filter. The filter may be arranged at the second inlet arrangement through which the air is introduced into the filtering chamber, or the filter may be located inside filtering chamber or in other place inside the inner space.

The inlet arrangements 6 for introducing contaminated air into the inlet chamber 5 and into the filtering chamber 11 may be provided at the same wall or plate.

For channeling the air into desired chamber, a control means may be provided. In flush mode, the air is flown from the filtering chamber 11 into the inlet chamber 5 and further to the outlet chamber 4 before it is discharged out of the unit. The filtering chamber 11 may be provided adjacent to the inlet chamber 5, and they may be separated by a second plate. The second plate may have at least one opening for allowing air flow from the filtering chamber 11 into the inlet chamber 5.

In sterilizing cycle, the second inlet arrangement 13 or inlet arrangements to the filtering chamber 11 may be closed for preventing air flowing into the filtering chamber. In flushing cycle, the inlet arrangement or inlet arrangements 6 allowing air flowing into the inlet chamber 5, without flowing first via filtering chamber 11, is closed. Channeling the air flow may be configured by control means. The optional control means may comprise at least one damper for channeling the air from the space either into the inlet chamber 5 or into the filtering chamber 11. The damper or the dampers may be provided at the inlet arrangement 6 allowing air suction into the inlet chamber 5, or it/they may be provided at the second inlet arrangement 13 allowing air suction into the filtering chamber 11. Optionally, the damper or dampers are arranged at each inlet arrangement(s) that allow air suction into the inlet chamber 5 and filtering chamber 11. Optionally, one common damper is provided at two inlet arrangements, e.g. a first inlet arrangement, for introducing air into the inlet chamber 5, and second inlet arrangement 13, for introducing air into the filtering chamber 11, for channeling the air from the space into the inlet chamber 5 or into the filtering chamber 11.

Optionally, the inner space 3 comprises an H₂O₂ generator chamber 15 which is arranged between the outlet chamber 4 and the inlet chamber 5. The H₂O₂ supply device 9 may be arranged inside the H₂O₂ generator chamber 15. In an embodiment having an H₂O₂ chamber, the first plate 8 is dividing the outlet chamber 4 and the H₂O₂ generator chamber 15. By having a separate H₂O₂ generator chamber 15 and providing the H₂O₂ supply device 9 inside the H₂O₂ generator chamber 15, it is ensured that the H₂O₂ chamber is kept under pressure to prevent unwanted leakage of H₂O₂ into the environment.

The fan 10 may be arranged inside the outlet chamber 4, and it may be arranged to suck air from the air chamber before the fan 10 in air flow direction. The fan 10 may be arranged run continuously with a predefined speed throughout the whole decontamination process, i.e. sterilization cycle and flushing cycle. The predefined speed may be constant at least during one of the cycles or it may be constant throughout the whole sterilization process. Optionally, the fan 10 speed may be adjusted.

The discharge arrangement may comprise a diffuser 16 for generating efficient forced diffusion of the H₂O₂-air mixture into the space. The diffuser 16 may be multi-diffuser type comprising multiple mini diffusers allowing multiple air diffusion patterns to be generated by adjusting the mini diffusers in different positions. For example, mini diffusers may be adjusted from fully vertical directed to angled, fully horizontal to radial, or 1, 2, 3, 4 -directional flow. By the arrangement, the flow pattern may be optimized to spaces of any shape. Optionally, the discharge arrangement comprises a grille or perforated plate.

Optionally, the discharge arrangement comprises at least one tube connector 17 for connecting the outlet chamber 4 into a ductwork of a building or to an opening in a wall of a room. This allows diffusing the H₂O₂-air mixture either directly into the space, or to a duct or to a tube for diffusing the H₂O₂-air to another space where the unit is located.

Optionally, the discharge arrangement comprises at least one clamp connector for connecting the outlet chamber 4 into an air channel, such as tube or a duct, for supplying the H₂O₂-air mixture from the decontamination unit 1 into the air channel. This arrangement allows the H₂O₂-air mixture being supplied to another space via air channel.

At least one of the inlet arrangements 6 of the decontamination unit 1 may comprise at least one opening for allowing suction of the air directly from the space into the decontamination unit 1, or at least one clamp connector for connecting the decontamination unit 1 into a tube or duct allowing suction of the air through the tube or duct. By having at least one clamp connector in the inlet arrangement 6, it is possible to connect a tube or a duct to the decontamination unit 1 and suck air from another contaminated space, located in different place than the unit, into the unit.

The decontamination unit 1 may comprise a control unit for controlling the unit operation. The control unit may be controlling the different cycles, sterilizing cycle and flushing cycle, of the unit. Additionally, or optionally, the control unit may be used for at least one of the following functions: control the fan 10 speed, monitor the H₂O₂ concentration level, monitor the H₂O₂ supply dose, adjust the H₂O₂ supply, and send command to HVAC systems. The control unit may be configured to monitor and/or control the H₂O₂ concentration in the space.

The decontamination unit 1 or the control unit may be connected to a building management system (BMS) for allowing automated use of the unit, using the unit at a distance, and/or co-operate with HVAC system of a building or such where the unit is located. For example, the unit may be connected to the BMS to send command to change the HVAC system control position to predefined sterilization mode (e.g. close ventilation of contaminated space), receive information from the BMS when the sterilization mode is activated to start decontamination process, send command to the BMS to change into predefined flush mode to remove H₂O₂ from the space, and/or send command (or information that decontamination process is completed) to the BMS to change the HVAC system to normal mode. The unit may be connected for example via BUS-connection.

The decontamination unit 1 may be mobile, or it may be arranged to be fixed to a building. The mobile decontamination unit 1 may be provided with wheels for moving the unit between different spaces, e.g. rooms.

The decontamination system may comprise two or more decontamination units as described herein. The decontamination units may be located in same space, or they may be located in different spaces for sterilizing several spaces one at a time or at the same time. The decontamination units may be connected to a building management system. The decontamination units may be connected to each other, e.g. via BUS-connection.

Figures 6a and 6b show an embodiment wherein the second inlet arrangement 13 is arranged to a separate plate, which is after the inlet arrangement in air flow direction, through which the contaminated air is introduced inside the decontamination unit 1. The separate plate may comprise also another inlet allowing suction of the air to be introduced into the inlet chamber 5 without flowing through the filtering chamber 11. The inlet for the inlet chamber 5 and the second inlet 13 for the filtering chamber 11 may be arranged next to each other and channeling the air through one of the inlet arrangements may be controlled by control means having e.g. damper. Figure 6a shows sterilizing cycle, wherein the air from the contaminated space is introduced into the decontamination unit 1 and flown through the inlet chamber 5, H₂O₂ generator chamber 15 and outlet chamber 4 before discharged back into the space. Figure 6b shows flushing cycle, wherein the air from the contaminated space is introduced into the decontamination unit 1 through the same inlet as in figure 6a, but the air is channeled via the second inlet arrangement 13to the filtering chamber 11 for capturing the H₂O₂ from the air before the inlet chamber 5.

The decontamination unit may comprise at least one sensor for measuring and monitoring H₂O₂ content in the air flow inside the unit. At least one sensor may be arranged inside the inlet chamber 5 so that the H₂O₂ content may be measured directly from the air flow inside the unit. Optionally or additionally, at least one sensor is arranged at the inlet, through which the air is introduced into the unit. Optionally, the unit comprises several sensors for measuring H₂O₂ content in the air. Optionally or additionally, the decontamination unit may comprise connector or connectors for connecting the unit to sensors located outside of the unit. One possible location for the sensor 30 is shown in figures 1b and 2b. However, sensor or sensors may be implemented in any embodiments described herein.

The decontamination system may comprise two or more decontamination units as described herein. The decontamination units may be located in same space, or they may be located in different spaces for sterilizing several spaces one at a time or at the same time. The decontamination units may be connected to a building management system. The decontamination units may be connected to each other, e.g. via BUS-connection.

The inlet and the outlet may comprise detachable inlet module and/or outlet module, respectively. The detachable modules may comprise different kind of inlet connections or openings, for introducing the air into the decontamination unit, or outlet connections or openings, for discharging the gas mixture out of the unit. Different modules may be used for connecting the unit to different kind of connections, such as air channels or openings in a wall, or discharging gas mixture directly into the space.

The following describes a method for sterilizing contaminated space by recycling air from the contaminated space through a decontamination unit 1. The decontamination unit may be any kind of unit described herein. The sterilizing process may be divided in two cycles, a sterilizing cycle and a flushing cycle. In the sterilizing cycle, H₂O₂ is introduced into the contaminated space for sterilizing the space. Once the sterilization cycle is completed, the H₂O₂ is removed from the space by the flushing cycle. In the sterilization cycle, the air from the contaminated space is recirculated through the decontamination unit 1 by a fan 10, having a fan inlet and a fan outlet, inside the unit. The fan 10 creates an air flow inside the unit from an inlet arrangement 6 to a discharge arrangement and the air is flown through an inlet chamber 5 and outlet chamber 4. H₂O₂ is supplied directly into the air flow by an H₂O₂ supply device 9 which is arranged next to the fan inlet or the fan outlet. The H₂O₂-air mixture is discharged into the contaminated space via the outlet. The air is recirculated until sufficient decontamination dose is supplied. The decontamination dose may be monitored manually or automatically. The decontamination unit 1 may comprise a H₂O₂ generation chamber arranged between the inlet chamber 5 and the outlet chamber 4, wherein the air is flowing through the H₂O₂ generation chamber.

Once sufficient decontamination dose is reached, a flushing cycle for reducing H₂O₂ concentration in the space may begin. In flush cycle, the H₂O₂ supply is closed and air from the space is circulated through a filtering chamber 11, inlet chamber 5 and outlet chamber 4 and back to the space via the outlet. The air to the filtering chamber 11 may be introduced via a second inlet 13 for allowing the air from the space circulate into the filtering chamber 11 before the inlet chamber 5. The H₂O₂ is captured from the recirculating air inside the filtering chamber 11. The H₂O₂ concentration in the space may be monitored and once desired level of concentration is reached at the end of the flushing cycle, the fan 10 is being shut down. The desired level may be for example less than 1 ppm.

Although the invention has been the described in conjunction with a certain type of device, it should be understood that the invention is not limited to any certain type of device. While the present inventions have been described in connection with a number of exemplary embodiments, and implementations, the present inventions are not so limited, but rather cover various modifications, and equivalent arrangements, which fall within the purview of prospective claims.

## Claims

1. A decontamination system for sterilizing a contaminated space, comprising at least one decontamination unit (1) through which the air of the contaminated space is recirculated, which unit comprises
- an inlet arrangement (6) for introducing air from the contaminated space into the unit,
- a discharge arrangement (7) for discharging gas out of the unit,
- a fan (9) for creating an air flow from the inlet arrangement to the discharging arrangement,
- H₂O₂ supply device (10) for introducing H₂O₂ into the air flow inside the unit,
ch a r a cte r i zed in that
the decontamination unit comprises at least one of
- the discharge arrangement comprises at least one detachable outlet module (21) through which the H₂O₂-air mixture is discharged out of the unit, and
- the inlet arrangement comprises at least one detachable inlet module (20) for receiving air from a space into the unit.

2. The decontamination system according to claim 1, wherein the discharge arrangement (7) comprises a detachable outlet module (21) comprising a diffuser.

3. The decontamination system according to claim 2, wherein the diffuser comprises multiple mini diffusers for controlling the diffuser pattern.

4. The decontamination system according to any one of claims 1 to 3, wherein the discharge arrangement comprises a detachable outlet module comprising at least one tube connector (17) for connecting the outlet chamber (4) into air channel, such as tube or duct, for supplying the H₂O₂-air mixture from the decontamination unit into the air channel.

5. The decontamination system according to any one of claims 1 to 4, wherein the discharge arrangement (7) comprises the detachable outlet module (21) comprising at least one clamp connector for connecting the unit to ductwork or a hole in wall of a space, which allows diffusing the H₂O₂-air mixture either directly into the space, or to a duct or tube.

6. The decontamination system according to claim 1, wherein the system comprises at least two detachable outlet modules of group: outlet module with diffuser, outlet module with tube connector, and outlet module with clamp connector.

7. The decontamination system according to any one of claims 1 to 6, wherein the inlet arrangement (6) comprises a detachable inlet module (20) comprising a grille or perforated plate.

8. The decontamination system according to any one of claims 1 to 7, wherein the inlet arrangement (6) comprises a detachable inlet module (20) comprising at least one tube connector for connecting the unit to an air channel, such as a tube or a duct, for receiving air via air channel from different location.

9. The decontamination system according to any one of claims 1 to 8, wherein the inlet arrangement (6) comprises a detachable inlet module (20) comprising at least one clamping ring for connecting the unit to ductwork or a hole in wall of a space, which allows receiving the air either directly from the space, or via a duct or tube.

10. The decontamination system according to any one of claims 1 to 9, wherein the inlet arrangement (6) comprises control means (14) for channeling the air in different air chambers inside the unit.

11. The decontamination system according to claim 10, wherein the control means (14) comprises a damper arranged at the at least two openings for controlling into which air chamber the air from the contaminated space is introduced.

12. The decontamination system according to any one of claims 1 to 11, wherein the inlet arrangement (6) comprises at least two openings, wherein at least two openings lead to different air chambers inside the unit.

13. The decontamination system according to any one of claims 1 to 12, wherein the system comprises tubing connected to the outlet module, which tubing ends to another space than the location of the decontamination unit.

14. The decontamination system according to any one of claims 1 to 13, wherein the system comprises tubing connected to the inlet module, which tubing ends to another space wherein the unit is located.

15. The decontamination system according to any one of claims 1 to 14, wherein the unit comprises an inlet chamber for receiving the air from the contaminated space and a filtering chamber for removing H₂O₂ from the air from the space.
